# EUROPÄISCHE PATENTSCHRIFT

(11) **EP 1 066 026 B1**
(45) Veröffentlichungstag und Bekanntmachung des Hinweises auf die Patenterteilung: **28.09.2005**
(21) Anmeldenummer: 99913258.2
(22) Anmeldetag: 16.03.1999
(51) Int. Cl.: A61K 7/50, A61K 7/00

(54) **FLÜSSIGE KÖRPERREINIGUNGSMITTEL**
LIQUID BODY CLEANSING AGENTS
DETERGENTS CORPORELS LIQUIDES

(30) Priorität: 25.03.1998 DE 19813057
(43) Veröffentlichungstag der Anmeldung: 10.01.2001
(73) Patentinhaber: Henkel Kommanditgesellschaft auf Aktien, 40589 Düsseldorf (DE)
(72) Erfinder: PUJOL GALIANO, Miracle, E-08329 Teia (ES); LAPENA, Margarita, E-08026 Barcelona (ES); SCHOLZ, Wolfhard, D-47829 Krefeld (DE); SCHOSSER, Gryta, D-47829 Krefeld (DE)
(86) Internationale Anmeldenummer: PCT/EP1999/001699
(87) Internationale Veröffentlichungsnummer: WO 1999/048473

(56) Entgegenhaltungen:
- EP-A- 0 759 291
- EP-A- 0 763 341
- DE-A- 4 203 490
- GB-A- 2 297 761
- US-A- 5 520 919
- US-A- 5 556 887

## Beschreibung

Die Erfindung betrifft flüssige Körperreinigungsmittel mit relativ niedrigem Gehalt an haut- und schleimhautverträglichen Tensiden, Verdickungsmitteln und darin solubilisierten Ölkomponenten, die eine spezielle Kombination von Konservierungsmitteln enthalten, die den Erhalt der Stabilität und Viskosität des Systems gewährleistet.

Flüssige Körperreinigungsmittel werden heute mit Kombinationen von oberflächenaktiven Stoffen formuliert, die ein sehr hohes Schäum- und Reinigungsvermögen aufweisen und daher in sehr niedriger Dosierung zum Einsatz kommen und auf diese Weise sehr hautschonend sind. Andererseits soll die Hautfreundlichkeit durch Einarbeitung von Ölkomponenten weiter gesteigert werden. Diese Ölkomponenten sowie Duftstoffe sollen klar solubilisiert sein, damit die Zusammensetzungen lagerstabil sind und nicht zur Separation neigen. Schließlich sollen die Systeme durch geeignete und preiswerte Mittel verdickt werden, damit die Zubereitungen besser mit der Hand auf der Haut verteilt werden können und nicht zwischen den Fingern hindurchrinnen.

Die genannten Aufgaben sind bei niedrigen Tensidgehalten nur schwer zu erfüllen und es werden oft Zusammensetzungen von labiler Stabilität erhalten, die bei Zusatz weiterer Komponenten, z.B. von Konservierungsmitteln, die für die mikrobiologische Stabilität erforderlich sind, leicht ihre Klarflüssigkeit oder ihre Viskosität oder beides verlieren.

Insbesondere bei solchen Körperreinigungsmittel, die als Tenside eine Kombination von Aniontensiden, Betaintensiden und nichtionischen Tensiden, Verdickungsmittel vom Typ der Polyalkylenglycol-difettsäureester und solublisierte Ölkomponenten enthalten, macht die mikrobiologische Konservierung große Probleme in bezug auf den Erhalt der Klarheit und Viskosität des Systems. Es wurde nunmehr eine spezielle Kombination von Konservierungsmitteln gefunden, die eine Stabilisierung solcher Zubereitungen ohne Einbuße an Klarheit oder Senkung der Viskosität ermöglicht. Eine Konservierungsmittelkombination der genannten Art kann e.g. der EP-A-0 763 341 entnommen werden.

Gegenstand der Erfindung sind daher flüssige Reinigungsmittel mit einer Viskosität von mehr als 500 mPa·s (20°C) und einem Gehalt von

| | |
|---|---|
| 4 - 10 Gew.-% | hautfreundlicher Tenside, bestehend aus einem Cremisch von Aniontensiden, Betaintensiden und nichtionischen Tensiden |
| 0,5 - 2 Gew.-% | eines Verdickungsmittels aus der Gruppe der Fettsäureester hochmolekularer Polyalkylenglycole oder Polyalkylenglycolether |
| 0,5 - 1 Gew.-% | solubilisierter Ölkomponenten und Duftstoffe |

dadurch gekennzeichnet, daß zur Konservierung und Stabilisierung der Viskosität

| | |
|---|---|
| 1 - 2 Gew.-% | einer Kombination von Natriumbenzoat, p-Hydroxybenzoesäureestern und Phenoxyethanol im Gewichtsverhältnis 1 : (0,5 - 2) : (0,5 - 2) |

darin enthalten ist.

Das Gemisch der hautfreundlichen Tenside setzt sich bevorzugt aus Aniontensiden (A), Betaintensiden (B) und nichtionischen Tensiden (C) im Gewichtsverhältnis (A) : (B) : (C) = 1 : (0,5 - 1) : (0,5 - 1) zusammen.

Als anionische Tenside sind bevorzugt Fettalkoholethersulfate in Form ihrer Natrium- oder Magnesiumsalze geeignet. Es können auch Gemische verschiedener Alkylethersulfate mit unterschiedlicher Alkylkettenlänge, unterschiedlichen Oxethylierungsgraden sowie unterschiedlichen Kationen eingesetzt werden. Ein bevorzugt geeignetes Aniontensid enthält z.B. ein Gemisch von C₁₂₋₁₄-alkyl- und Oleyl-ethersulfaten und in Form eines Gemisches von Natrium- und Magnesiumsalzen. Ein solches, besonders haut- und schleimhautverträgliches Produkt ist unter der Bezeichnung Texapon® ASV (Henkel KGaA) im Handel.

Neben den genannten Fettalkoholethersulfaten können in geringerem Umfange von z.B. nicht mehr als 30 Gew.-% der Aniontenside auch andere anionische Tenside, z.B. Acylisethionate, Alkansulfonate, Acylsarkoside, Acyltauride und Acylmonoglyceridsulfate mit 12 -16 C-Atomen in der Acylgruppe in Form ihrer Natriumsalze enthalten sein.

Als Betaintenside sind bevorzugt Alkyl-(C₁₀-C₁₆)-dimethyl-acetobetaine oder Acyl-(C₁₀-C₁₆)-amidopropyl-dimethyl-acetobetaine enthalten. Geeignete Handelsprodukte dieses Typs sind z.B. Dehyton® AB 30, Dehyton® K und Dehyton® PK 45 (Cocoamidopropylbetain). Daneben können in geringerem Umfange, z.B. von nicht mehr als 30 Gew.-% der Betaintenside auch andere Betaintenside, z.B. Sulfobetaine oder Imidazolinbetaine enthalten sein.

Als nichtionogene Tenside eignen sich alle nichtionischen Tenside, die bei 20°C zu wenigstens 5 Gew.-% klar wasserlöslich sind. Dies sind vor allem die Ethylenoxid-Anlagerungsprodukte an Fettsäuren, an Fettsäureglyceride, an Fettsäuresorbitanester, an Fettsäurealkanolamide oder an Methylglucosid-Fettsäureester. Sehr gut eignen sich aber auch die Alkyl-(oligo)-glucoside, die ein besonders gutes Solubilisierungsvermögen aufweisen.

Die Alkyl-(oligo)-glycoside, ihre Herstellung und Verwendung als oberflächenaktive Stoffe sind z.B. aus US-A-3,839,318, DE-A-20 36 472, EP-A-77 167 oder WO-A-93/10132 bekannt. Bezüglich des Glycosidrestes gilt, daß sowohl Monoglucoside, bei denen ein Monosaccharid glycosidisch an einen Fettalkohol mit 10 - 16 C-Atomen gebunden ist, als auch oligomere Glucoside mit einem Oligomerisationsgrad bis 10 geeignet sind. Der Oligomerisationsgrad ist dabei ein statistischer Mittelwert, dem eine für solche technischen Produkte übliche Homologenverteilung zugrunde liegt. Bevorzugt eignen sich als Alkyl-(oligo)-glycoside solche der Formel RO(C₆H₁₀O)ₓH, in der R eine Alkylgruppe mit 12 bis 14 C-Atomen ist und x einen Mittelwert von 1 bis 2 hat. Geeignete Produkte sind z.B. unter der Bezeichnung Plantacare®2000 am Markt.

Eine weitere, bevorzugte Gruppe von nichtionischen Tensiden sind die Fettsäure-C₈-C₁₈-mono- und -diester von Anlagerungsprodukten von 4 - 20 Mol Ethylenoxid an Glycerin, wie sie z.B. in DE 20 24 051 als Rückfettungsmittel beschrieben sind. Solche Produkte sind unter der Bezeichnung Cetiol®HE im Handel.

Als Verdickungsmittel eignen sich besonders die Fettsäureester von hochmolekularen Polyalkylenglycolen und Polyalkylenglycolethern. Insbesondere eignen sich Fettsäurediester von Polyethylenglycolen aus 50 - 200 Glycolethergruppen oder Fettsäureester von Alkyl-C₁₈-C₂₂-polyethylenglycolethern mit 50 - 200 Glycolethergruppen. Bevorzugt ist als Verdickungsmittel ein Polyethylenglycol-distearat enthalten, dessen Polyethylenglycol-Teil aus 100 - 200 Glycolethergruppen besteht. Ein geeignetes Handelsprodukt ist z.B. Eumulgin® EO33 (PEG 150 Distearate).

Die erfindungsgemäßen flüssigen Reinigungsmittel enthalten solubilisierte Ölkomponenten und Duftstoffe in einer Menge von 0,5 - 1 Gew.-%. Als Ölkomponenten können dabei alle bekannten hautweichmachenden Öle, z.B. Paraffinöle, Isoparaffine, 1,3-Dialkylcyclohexan, 2-Hexyldecanol, 2-Octyl-dodecanol, Di-n-octylether, Fettsäureester wie z.B. Isopropylmyristat, Isopropylstearat, Decyloleat, n-Hexyllaurat, Oleylerucat, Cetyloleat und andere bekannte Ölkomponenten sowie ätherische Öle oder synthetische Duftstoffe enthalten sein. Bevorzugt sind als solubilisierte Ölkomponenten Fettsäureester, Parfümöle oder Gemische davon enthalten.

Die erfindungsgemäß enthaltene Kombination von Konservierungsstoffen enthält als p-Hydroxybenzoesäureester bevorzugt ein Gemisch aus dem wasserlöslichen Methylester (M) und dem öllöslichen Propylester (P) im Gewichtsverhältnis (M) : (P) = 1 : (0,2 - 1).

Darüber hinaus können die erfindungsgemäßen Reinigungsmittel übliche wasserlösliche Zusätze in kleineren Mengen, die der Stabilität des Systems nicht abträglich sind, enthalten. Als Beispiele für solche Zusätze seien genannt: Farbstoffe zur Einfärbung der Produkte, Citronensäure zur pH-Einstellung, wasserlösliche Proteine bzw. Proteinhydrolysate, wasserlösliche Polymere, wasserlösliche Pflanzenextrakte sowie ggf.

Elektrolyte. Bevorzugt ist ein anorganischer Elektrolyt, z.B. Natriumchlorid oder Magnesiumchlorid in einer Menge von 0,1 - 1 Gew.-% zur Verdickung und Viskositätsstabilisierung enthalten.

Das Herstellungsverfahren für die erfindungsgemäßen Körpenreinigungsmittel hat ebenfalls einen erheblichen Einfluß auf die klare und homogene Solubilisierung der Ölkomponenten. Dabei hat es sich als vorteilhaft erwiesen, daß man die wasserunlöslichen Ölkomponenten, die öllöslichen Konservierungsmittel und Duftstoffe mit den nichtionogenen Tensiden mischt und die Mischung unter Rühren erwärmt, bis sich eine homogene Lösung gebildet hat. In diese Lösung gibt man dann die wäßrigen Zubereitungen der anionischen und betainischen Tenside, die ggf. auf die gleiche Temperatur erwärmt wurde, unter Rühren ein. Schließlich werden die wasserlöslichen Konservierungsmittel sowie gegebenenfalls andere wasserlösliche Hilfsmittel zugegeben und eingemischt.

Die Herstellung der in den erfindungsgemäßen Beispielen 1 - 3 genannten Rezepturen kann z.B. kontinuierlich aus folgenden separat vorbereiteten Vorgemischen erfolgen:
- Phase 1 :: Man löst PHB-Propylester in Cetiol HE und fügt nacheinander Isopropylmyristat, Duftstoff und ggf. Farbstoff in einer geringen Menge Wasser hinzu.
- Phase 2 :: Man löst Natriumbenzoat in einem Teil des Wassers bei 80 - 85° C, löst den PHB-Methylester, die Hälfte des Eumulgin EO33 unter starkem Rühren bei 85°C, gibt Wasser von 20°C zur Kühlung zu, fügt dann Plantacare 2000, Texapon ASV und Phenoxyethanol zu.
- Phase 3 :: Man erhitzt Wasser auf 80 - 85°C und löst darin den Rest des Eumulgin EO33, löst darin eine kleine Menge (0,6 Gew.-%) Texapon ASV und gibt Wasser von 20°C zur Kühlung zu.
- Phase 4 :: In einem kontinuierlichen Mischaggregat (z.B. Conti Bran-Lubbe-Mischer) werden Phase 3, Phase 2, Phase 1, NaCl-Lösung, Wasser, Dehyton PK45 und ggf. Zitronensäurelösung zur pH-Einstellung miteinander gemischt.

Die folgenden Beispiele sollen den Gegenstand der Erfindung näher erläutern:

### Beispiele

Es wurden die folgenden Reinigungsmittel hergestellt:

| Rezepturen | 1 | 2 | 3 | V1 | V2 |
|---|---|---|---|---|---|
| Texapon® ASV (1) | 10 | 15 | 15 | 10 | 10 |
| Dehyton® PK 45 (2) | 5 | 7 | 8 | 4,6 | 4,6 |
| Plantacare® 2000 (3) | 2,0 | 4,0 | 5,0 | 2,0 | 2,0 |
| Gluadin® W 40 (4) | - | - | - | 1,25 | 1,25 |
| Cetiol® HE (5) | 1,0 | 1,0 | 2,0 | 0,9 | 0,9 |
| Isopropylmyristat | 0,5 | 0,8 | 0,6 | 0,45 | 0,45 |
| Oleylerucat | - | - | - | 0,1 | 0,1 |
| Parfüm | 0,3 | 0,3 | 0,3 | 0,3 | 0,3 |
| Cremophor® RH 60 (6) | - | - | - | 0,1 | 0,1 |
| Natriumbenzoat | 0,5 | 0,5 | 0,4 | 0,4 | 0,55 |
| PHB-Methylester | 0,2 | 0,3 | 0,1 | - | - |
| PHB-Propylester | 0,1 | 0,1 | 0,05 | - | - |
| Phenoxyethanol | 0,5 | 0,8 | 0,7 | - | - |
| Euxyl K-400 | - | - | - | 0,2 | - |
| Eumulgin® EO 33 (7) | 1,3 | 1,5 | 1,0 | 1,3 | - |
| NaCl | 0,3 | 0,3 | 0,3 | 0,3 | - |
| Carbopol® ETD 2020 (9) | - | - | - | - | 0,6 |
| Wasser | ad 100 | ad 100 | ad 100 | ad 100 | ad 100 |
| Aussehen | klar | klar | klar | klar | trüb |
| pH-Wen | 4,8 | 4,9 | 4,8 | 4,7 | 4,7 |
| Viskosität (20°C) [mPa·s] nach 24 Std. Lagerung | 3400 | 2600 | 1400 | 600 | 1000 |
| Viskosität (20°C) [mPa·s] 56 Tage Lagerung (45°C) | 2100 | 2000 | 1300 | < 50 | 900 |

Es wurden die folgenden Handelprodukte verwendet:

| | | |
|---|---|---|
| (1) | Texapon® ASV | Gemisch aus Laurylethersulfaten mit 2 EO und 8 EO und Oleylethersulfat mit 2 EO in Form von Natrium- und Magnesiumsalzen (30 %ig in Wasser) |
| (2) | Dehyton® PK 45 | Cocoamidopropyl-betaine (40 %ig in Wasser) |
| (3) | Plantacare® 2000 | C₈-C₁₆-Alkyl-(1,4)-glucosid (50 %ig in Wasser) |
| (4) | Gluadin® W 40 | Weizenproteinhydrolysat |
| (5) | Cetiol®HE | Glycerinpolyglycolether-(7 EO)-kokosfettsäuremonoester |
| (6) | Cremophor® RH 60 | Hydr. Rizinusöl-polyglycolether (60 EO) |
| (7) | Eumulgin® EO 33 | Polyethylenglycol (150 EO)-distearat |
| (8) | Natrosol® 250 HR | Hydroxyethylcellulose (1 %ig in Wasser, 1500 - 2500 mPa·s (20°C)) |
| (9) | Carbopol® ETD 2020 | Polyacrylsäure-Copolymer |

Die Viskosität wurde mit einem Rotationsviskosimeter (Brookfield, Typ RVT, Spindel 2 bei 20 UPM) bestimmt.

## Patentansprüche

1. Flüssige, wäßrige Reinigungsmittel mit einer Viskosität von mehr als 500 mPa·s (20°C) und mit einem Gehalt von
| | |
|---|---|
| 4 - 10 Gew.-% | hautfreundlicher Tenside, bestehend aus einem Gemisch von Aniontensiden, Betaintensiden und nichtionischen Tensiden |
| 0,5 - 2 Gew.-% | eines Verdickungsmittels aus der Gruppe der Fettsäureester hochmolekularer Polyalkylenglycole oder Polyalkylenglycolether |
| 0,5 - 1 Gew.-% | solubilisierter Ölkomponenten und Duftstoffe |
**dadurch gekennzeichnet, daß** zur Konservierung und Stabilisierung der Viskosität
| | |
|---|---|
| 1 - 2 Gew.-% | einer Kombination von Natriumbenzoat, p-Hydroxybenzoesäureestern und Phenoxyethanol im Gewichtsverhältnis 1 : (0,5 - 2) : (0,5 - 2) |
enthalten ist.

2. Reinigungsmittel gemäß Anspruch 1, **dadurch gekennzeichnet, daß** als Aniontensid ein Gemisch von C_{12/14}-alkyl- und Oleyl-Ethersulfaten in Form eines Gemisches von Natrium- und Magnesiumsalzen enthalten ist.

3. Reinigungsmittel nach einem der Ansprüche 1 oder 2, **dadurch gekennzeichnet, daß** als Verdickungsmittel ein Polyethylenglycol-distearat, dessen Polyethylenglycol-Teil aus 100 - 200 Glycolethergruppen besteht, enthalten ist.

4. Reinigungsmittel nach einem der Ansprüche 1 - 3, **dadurch gekennzeichnet, daß** als solubilisierte Ölkomponenten Fettsäureester, Parfümöle oder Gemische davon enthalten sind.

5. Reinigungsmittel nach einem der Ansprüche 1 - 4, **dadurch gekennzeichnet, daß** als p-Hydroxybenzoesäureester ein Gemisch aus dem Methylester und dem Propylester im Gewichtsverhältnis 1 : (0,2 - 1) enthalten ist.

6. Reinigungsmittel nach einem der Ansprüche 1 - 5, **dadurch gekennzeichnet, daß** ein anorganischer Elektrolyt, bevorzugt Natriumchlorid oder Magnesiumchlorid, in einer Menge von 0,1 - 1 Gew.-% enthalten ist.

## Claims

1. Liquid water-based cleansing preparation with a viscosity of more than 500 mPas (20°C) containing
| | |
|---|---|
| 4 to 10% by weight | of skin-friendly surfactants consisting of a mixture of anionic surfactants, betaine surfactants and nonionic surfactants, |
| 0.5 to 2% by weight | of a thickener from the group of fatty acid esters of high molecular weight polyalkylene glycols or polyalkylene glycol ethers, |
| 0.5 to 1 % by weight | of solubilized oil components and perfumes, |
**characterized in that** the preparation also contains
| | |
|---|---|
| 1 to 2% by weight | of a combination of sodium benzoate, p-hydroxybenzoic acid esters and phenoxyethanol in a ratio by weight of 1 : (0.5-2) : (0.5-2) |
as a preservative and viscosity stabilizer.

2. A cleansing preparation as claimed in claim 1, **characterized in that** a mixture of C_{12/14} alkyl and oleyl ether sulfates in the form of a mixture of sodium and magnesium salts is present as the anionic surfactant.

3. A cleansing preparation as claimed in claim 1 or 2, **characterized in that** it contains a polyethylene glycol distearate, of which the polyethylene glycol part consists of 100 to 200 glycol ether groups, as the thickener.

4. A cleansing preparation as claimed in any of claims 1 o 3, **characterized in that** it contains fatty acid esters, perfume oils or mixtures thereof as the solubilized oil components.

5. A cleansing preparation as claimed in any of claims 1 to 4, **characterized in that** it contains a mixture of the methyl ester and the propyl ester in a ratio by weight of 1 : (0.2 - 1) as the p-hydroxybenzoic acid ester.

6. A cleansing preparation as claimed in any of claims 1 to 5, **characterized in that** an inorganic electrolyte, preferably sodium chloride or magnesium chloride, is present in a quantity of 0.1 to 1% by weight.

## Revendications

1. Produits de nettoyage liquides aqueux ayant une viscosité supérieure à 500 mPas (20°C) et contenant
| | |
|---|---|
| 4 - 10 % en poids | d'agents tensioactifs doux pour la peau, constitués d'un mélange d'agents tensioactifs anioniques, d'agents tensioactifs de type bêtaïne et d'agents tensioactifs non ioniques, |
| 0,5 - 2 % en poids | d'un épaississant du groupe des esters d'acides gras de polyalkylèneglycols ou d'éthers de polyalkylèneglycols à haut poids moléculaire, |
| 0,5 - 1 % en poids | de composants huileux et de parfums solubilisés, |
**caractérisés en ce qu'**y y sont contenus, aux fins de conservation et de stabilisation de la viscosité
| | |
|---|---|
| 1 - 2 % en poids | d'une combinaison de benzoate de sodium, d'esters de l'acide p-hydroxybenzoïque et de phénoxyéthanol dans un rapport pondéral de 1 : (0,5 - 2) : (0,5 - 2). |

2. Produits de nettoyage selon la revendication 1, **caractérisés en ce qu'**y y est contenu comme agent tensioactif anionique un mélange d'éthersulfates d'alkyle en C₁₂ à C₁₄ et d'oléyle sous la forme d'un mélange de sels de sodium et de magnésium.

3. Produits de nettoyage selon l'une des revendications 1 ou 2, **caractérisés en ce qu'**y y est contenu comme épaississant un distéarate de polyéthylèneglycol, dont la partie polyéthylèneglycol se compose de 100 à 200 groupes éther de glycol.

4. Produits de nettoyage selon l'une des revendications 1 - 3, **caractérisés en ce qu'**y y sont contenus comme composants huileux solubilisés des esters d'acides gras, des huiles parfumées ou leurs mélanges.

5. Produits de nettoyage selon l'une des revendications 1 - 4, **caractérisés en ce qu'**y y est contenu comme ester de l'acide p-hydroxybenzoïque un mélange de l'ester méthylique et de l'ester propylique dans un rapport pondéral de 1 : (0,2 - 1).

6. Produits de nettoyage selon l'une des revendications 1 - 5, **caractérisés en ce qu'**y y est contenu un électrolyte inorganique, de préférence le chlorure de sodium ou le chlorure de magnésium, en une quantité de 0,1 - 1 % en poids.
